# EUROPEAN PATENT SPECIFICATION

(11) **EP 3 366 779 B1**
(45) Date of publication and mention of the grant of the patent: **07.06.2023**
(21) Application number: 16857119.8
(22) Date of filing: 16.03.2016
(51) Int. Cl.: C12Q 1/02, C12N 5/071, C12N 5/0735, C12N 5/10, C12N 5/074

(54) **EVALUATION METHOD FOR DIFFERENTIATION STATE OF CELLS**
BEWERTUNGSVERFAHREN ZUR DIFFERENZIERUNG DES ZUSTANDS VON ZELLEN
MÉTHODE D'ÉVALUATION DE L'ÉTAT DE DIFFÉRENCIATION DE CELLULES

(30) Priority: 23.10.2015 WO PCT/JP2015/080003
(43) Date of publication of application: 29.08.2018
(73) Proprietor: Shimadzu Corporation, Kyoto-shi, Kyoto 604-8511 (JP); Tokyo Electron Limited, Tokyo 107-6325 (JP)
(72) Inventor: SUZUKI, Takashi, Kyoto-shi Kyoto 604-8511 (JP); HIRAMARU, Daisuke, Kyoto-shi Kyoto 604-8511 (JP); TAKAHASHI, Masatoshi, Kyoto-shi Kyoto 604-8511 (JP); TOMITA, Kentaro, Tokyo 107-6325 (JP); HATABAYASHI, Kunitada, Tokyo 107-6325 (JP); KAGAWA, Kenichi, Tokyo 107-6325 (JP); OZAKI, Shigenori, Nirasaki City Yamanashi 407-0192 (JP)
(74) Representative: Müller-Boré & Partner Patentanwälte PartG mbB
(86) International application number: PCT/JP2016/058303
(87) International publication number: WO 2017/068801

(56) References cited:
- EP-A1- 3 138 922
- WO-A1-2013/065302
- WO-A1-2014/188994
- WO-A1-2014/200068
- WO-A1-2015/166845
- WO-A1-2016/052558
- GABRIELA G. CEZAR ET AL: "Identification of Small Molecules from Human Embryonic Stem Cells Using Metabolomics", STEM CELLS AND DEVELOPMENT, vol. 16, no. 6, 1 December 2007 (2007-12-01), pages 869-882, XP055559282, ISSN: 1547-3287, DOI: 10.1089/scd.2007.0022
- PAUL RAMM SANDER ET AL: "Stem cell metabolic and spectroscopic profiling", TRENDS IN BIOTECHNOLOGY., vol. 31, no. 3, 1 March 2013 (2013-03-01), pages 204-213, XP055559236, GB ISSN: 0167-7799, DOI: 10.1016/j.tibtech.2013.01.008
- MARTA M. SILVA ET AL: "Robust Expansion of Human Pluripotent Stem Cells: Integration of Bioprocess Design With Transcriptomic and Metabolomic Characterization : hPSC Expansion Bioprocess and "Omics" Tools", STEM CELLS TRANSLATIONAL MEDICINE, vol. 4, no. 7, 15 May 2015 (2015-05-15), pages 731-742, XP055559262, US ISSN: 2157-6564, DOI: 10.5966/sctm.2014-0270
- RAED ABU DAWUD ET AL: "Human Embryonic Stem Cells and Embryonal Carcinoma Cells Have Overlapping and Distinct Metabolic Signatures", PLOS ONE, vol. 7, no. 6, 29 June 2012 (2012-06-29), page e39896, XP055395796, DOI: 10.1371/journal.pone.0039896
- TAKASHI SUZUKI ET AL.: 'Development of Technology for Quality Evaluation of Human Pluripotent Stem Cells by Metabolome Analysis' SHIMADZU REVIEW vol. 70, no. 3-4, 31 March 2014, pages 123 - 131, XP055377192
- TAKASHI SUZUKI ET AL.: 'Ekitai Chromatograph Shitsuryo Bunsekikei ni yoru Tanosei Kansaibo no Baiyojosei Seibun Issei Bunsekikei no Kaihatsu to sono Oyo' REGENERATIVE MEDICINE vol. 14, 01 February 2015, page 318
- PANOPOULOS AD. ET AL.: 'The metabolome of induced pluripotent stem cells reveals metabolic changes occurring in somatic cell reprogramming.' CELL RES vol. 22, no. 1, January 2012, pages 168 - 177, XP055377197

## Description

The present invention relates to a method for evaluating a differentiation state of cells.

Conventionally, in order to evaluate a differentiation state of cells, a method in which immunostaining is used (for example, see Patent Document 1) and a method in which an expression level of a marker gene is quantified (for example, see Patent Document 2) are widely used.

In the method in which immunostaining is used, first, cells, for example, pluripotent stem cells, which are to be evaluated, are immobilized using paraformaldehyde or the like, and then, are subjected an antigen-antibody reaction. Here, SSEA-4 or TRA1-60 is widely used as an antibody for determining whether or not the pluripotent stem cells are in an undifferentiated state (for example, see Patent Document 1). Subsequently, a secondary antibody that binds to the antibody is added to the cells, and then a fluorescent label or the like attached to the secondary antibody in advance is detected. As a result, whether or not an antigen to the antibody exists on the cells, that is, whether or not the cells are in an undifferentiated state can be evaluated.

Further, in the method based on quantification of an expression level of a marker gene, for example, mRNA is extracted from the pluripotent stem cells and is converted to cDNA using reverse transcriptase, and then, the marker gene is amplified by a PCR (polymerase chain reaction). In this case, NANOG or POU5F1 (OCT 3/4) is widely used as a marker gene for evaluating an undifferentiated property of the pluripotent stem cells (for example, see Non-Patent Document 1). By detecting a product of the PCR using electrophoresis or a real time PCR device, the expression level of the marker gene in the cells is confirmed, and, from the result, whether or not the cells are in an undifferentiated state is evaluated.
Patent Literature 1: Japanese Patent Laid-Open Publication No. 2004-313184.
Patent Document 2: Japanese Patent Laid-Open Publication No. 2006-042663.
Non-Patent Document 1: Nature Biotechnology, 2007, Volume 25, pages 803 - 816.

However, in all of the above-described conventional evaluation methods, it is necessary to perform an invasive treatment to the cells. Therefore, after evaluating a differentiation state, cells subjected to the evaluation could not be used for other purposes, for example, as a cell source for regenerative medicine. Further, for the same sample (that is, cells in the same culture dish), it was not possible to evaluate change with time. In order to evaluate temporal change of a differentiation state, complicated work, such as culturing multiple culture dishes in parallel, was necessary.

The present invention is accomplished in view of the above problem and is intended to provide a method for noninvasively evaluating a differentiation state of cells.

As a result of intensive studies, the present inventors found that an abundance of a specific indicator substance in a culture supernatant varies depending on a differentiation state of cells, and thus accomplished the present invention.

In the cell differentiation state evaluation method according to the present invention, stem cells for which a differentiation state is unknown, or cells that are differentiation-induced from pluripotent stem cells are used as test cells, and pluripotent stem cells are used as control cells, a differentiation state of the test cells is evaluated by comparing abundances of indicator substances in a culture supernatant of the test cells with abundances of the indicator substances in a culture supernatant of the control cells.

The indicator substances are two or more kinds of compounds selected from a group that consists of ornithine, 2-aminoadipic acid, deoxycytidine, tryptophan, hypoxanthine, uridine and kynurenine.

In the cell differentiation state evaluation method according to the present invention a differentiation state of test cells is evaluated by comparing abundances of indicator substances in a culture supernatant of the test cells with abundances of the indicator substances in a culture supernatant of control cells.

For example, for an indicator substance such as arginine, tryptophan, or 4-aminobenzoic acid, an abundance in a culture supernatant of differentiated cells tends to be larger than an abundance in a culture supernatant of undifferentiated cells. Therefore, where cells that are pluripotent stem cells are used as control cells, when a ratio of an abundance of an indicator substance such as arginine, tryptophan or 4-aminobenzoic acid in a culture supernatant of test cells to an abundance of the indicator substance in a culture supernatant of the control cells is equal to or greater than a predetermined threshold, it can be determined that the test cells are in a differentiated state.

For example, for an indicator substance such as ornithine, alanine, aspartic acid, 2-hydroxybutyric acid, 2-hydroxyisovaleric acid, urea, or 4-hydroxybenzoic acid, an abundance in a culture supernatant of differentiated cells tends to be smaller than an abundance in a culture supernatant of undifferentiated cells. Therefore, where pluripotent stem cells are used as control cells, when a ratio of an abundance of an indicator substance such as ornithine, alanine, aspartic acid, 2-hydroxybutyric acid, 2-hydroxyisovaleric acid, urea, or 4-hydroxybenzoic acid in a culture supernatant of test cells to an abundance of the indicator substance in a culture supernatant of the control cells is equal to or less than a predetermined threshold, it can be determined that the test cells are in a differentiated state.

According to the present invention, a differentiation state is evaluated based on abundances of two or more kinds of the above-described indicator substances. The differentiation state is evaluated by comparing, for each of two or more kinds of indicator substances, an abundance of an indicator substance in a culture supernatant of the test cells with an abundance of the indicator substance in a culture supernatant of the control cells. It is also possible to obtain, for each of two or more kinds of indicator substances, a ratio of an abundance on an indicator substance in a culture supernatant of the test cells to an abundance of the indicator substance in a culture supernatant of the control cells, and to evaluate the differentiation state based on whether or not these ratios are equal to or greater than a predetermined threshold. As the control cells, pluripotent stem cells are used.

As two or more kinds of indicator substances, for example, a precursor and a metabolic product in an anteroposterior relation of a metabolic pathway can be selected, and the differentiation state can be evaluated based on a ratio of an abundance of the precursor to the metabolic product in a culture supernatant of the test cells. Examples of a combination of a precursor and a metabolic product include a combination of tryptophan and kynurenine, a combination of ornithine and putrescine, a combination of arginine and ornithine, and the like.

As two or more kinds of indicator substances, for example, a combination including kynurenine, 2-aminoadipic acid, ornithine and deoxycytidine can be adopted. Two or more kinds of indicator substances may further include tryptophan, cystathionine, hypoxanthine, and uridine.

By evaluating the differentiation state based on abundances of two or more kinds of indicator substances, the differentiation state of the test cells can be evaluated with higher accuracy. Further, by evaluating the differentiation state based on abundances of two or more kinds of indicator substances, in addition to evaluation of whether or not the test cells are in a differentiated state or in an undifferentiated state, it is also possible to evaluate whether a differentiation direction of the test cells that have been evaluated as being in a differentiated state is endoderm, mesoderm, or ectoderm.

For example, by analyzing changes with culture time of abundances of two or more kinds of indicator substances selected from a group consisting of kynurenine, 2-aminoadipic acid and deoxycytidine in a culture supernatant of the test cells, whether or not the test cells are in a differentiated state or in an undifferentiated state can be evaluated, and whether a differentiation direction of the test cells that have been evaluated as being in a differentiated state is endoderm, mesoderm, or ectoderm can be evaluated.

By subjecting abundances of two or more kinds of indicator substances selected from a group consisting of kynurenine, ornithine, 2-aminoadipic acid, deoxycytidine, tryptophan, hypoxanthine, and uridine in a culture supernatant of the test cells to multivariate analysis, whether or not the test cells are in a differentiated state or in an undifferentiated state can be evaluated, and whether a differentiation direction of the test cells that have been evaluated as being in a differentiated state is endoderm, mesoderm, or ectoderm can be evaluated.

In the cell differentiation state evaluation method according to the present invention, the stem cells may be pluripotent stem cells such as ES cells (embryonic stem cells) or iPS cells (induced pluripotent stem cells).

The abundances of the indicator substances in the culture supernatant used in the cell differentiation state evaluation method according to the present invention may be obtained using any method. Examples of representative methods include a liquid chromatography analysis method and a mass spectrometry method.

According to the cell differentiation state evaluation method according to the present invention, a differentiation state of cells can be noninvasively evaluated without the need of destroying the cells as in a conventional method. As a result, after the evaluation of the differentiation state, it is possible to use the test cells as a cell source or the like for regenerative medicine. Further, even in a case of evaluating temporal change of the differentiation state, it is not necessary to perform complicated work such as culturing multiple culture dishes in parallel as in a conventional method, and it is possible to easily evaluate change with time of the differentiation state with cells in the same culture dish as targets. Further, by performing evaluation based on abundances of two or more kinds of indicator substances in the culture supernatant, the differentiation state of the test cells can be evaluated with higher accuracy. By performing evaluation based on abundances of two or more kinds of indicator substances, in addition to evaluation of whether or not the test cells are in a differentiated state or in an undifferentiated state, it is also possible to evaluate directionality of differentiation of the test cells that have been evaluated as being in a differentiated state.

### Brief Description of Drawings

[Fig. 1A] A schematic diagram describing a cell differentiation state evaluation method in an example of the present invention (Part 1).
[Fig. 1B] A schematic diagram describing the cell differentiation state evaluation method in the example of the present invention (Part 2).
[Fig. 2A] Graphs showing temporal changes of abundances of substances obtained by LC-MS analysis of culture supernatants in the example (Part 1).
[Fig. 2B] Graphs showing temporal changes of abundances of substances obtained by LC-MS analysis of culture supernatants in the example (Part 2).
[Fig. 2C] Graphs showing temporal changes of abundances of substances obtained by LC-MS analysis of culture supernatants in the example (Part 3).
[Fig. 2D] Graphs showing temporal changes of abundances of substances obtained by LC-MS analysis of culture supernatants in the example (Part 4).
[Fig. 3A] Graphs showing temporal changes of abundances of substances obtained by GC-MS analysis of culture supernatants in the example (Part 1).
[Fig. 3B] Graphs showing temporal changes of abundances of substances obtained by GC-MS analysis of culture supernatants in the example (Part 2).
[Fig. 4A] Graphs showing results of principal component analysis of abundances of substances in samples of Day 2 - Day 4 of culture in the example (Part 1).
[Fig. 4B] Graphs showing results of principal component analysis of abundances of substances in samples of Day 2 - Day 4 of culture in the example (Part 2).

In a cell differentiation state evaluation method according to the present invention, a differentiation state of test cells is evaluated by comparing abundances of two or more kinds of biomarkers (indicator substances) in a culture supernatant of the test cells and control cells.

As the test cells, stem cells, typically pluripotent stem cells such as ES cells or iPS cells can be used. Further, cells differentiation-induced from the stem cells can also be used as test cells. When stem cells for which a differentiation state is unknown are test cells, whether or not the test cells are in a differentiated state or in an undifferentiated state can be determined using the evaluation method of the present invention. When cells differentiation-induced from stem cells are test cells, whether or not cells in an undifferentiated state are mixed can be determined using the evaluation method of the present invention.

As a culture medium used for culturing test cells, a culture medium such as DMEM/F12 that is generally used for culturing stem cells, or a culture medium (such as mTeSR1, and TeSR-E8) containing the DMEM/F12 as a main component can be used. Table 1 shows components of DMEM/F12.

**[Table 1]**

| Components |
|---|
| **Amino Acids** |
| Glycine |
| L-Alanine |
| L-Arginine hydrochloride |
| L-Asperagine-H2O |
| L-Aspertic acid |
| L-Cysteine hydrochloride-H2O |
| L-Cystine 2HCl |
| L-Glutamic Acid |
| L-Glutamine |
| L-Histidine hydrochloride-H2O |
| L-Isoleucine |
| L-Leucine |
| L-Lysine hydrochloride |
| L-Methionine |
| L-Phenylalanine |
| L-Proline |
| L-Se rire |
| L-Threo nine |
| L-Tryptophan |
| L-Tyrosirie disodium salt dihydrate |
| L-Valine |

| **Vitamins** |
|---|
| Biotin |
| Choline chloride |
| D-Calcium pantothenate |
| Folic Acid |
| Niacinamide |
| Pyridoxine hydrochloride |
| Riboflavin |
| Thiamine hydrochloride |
| Vitamin 812 |
| i-Inositol |

| **Inorganic Salts** |
|---|
| Calcium Chloride (CaCl2) (anhyd) |
| Cupric sulfate (CuSO4-5H2O) |
| Ferric Nitrate (Fe(NO3)3"9H2O) |
| Ferric sulfate (FeSO4-7H2O) |
| Magnesium Chloride (anhydrous) |
| Magnesium Sulfate (MgSO4)(anhyd.) |
| Potassium Chloride (KCl) |
| Sodium Bicarbonate (NaHCO3) |
| Sodium Chloride (NaC!) |
| Sodium Phosphate dibasip (Na2HPO4) anhydrous |
| Sodium Phosphate monobasic(NaH2PO4-H2O) |
| Zinc sulfate (ZrSO4-7H2O) |

| **Others** |
|---|
| D-Glucose (Dextrose) |
| Hypoxanthine Na |
| Linoleic Acid |
| Lipic Acid |
| Phenol Red |
| Putrescine 2HCl |
| Sodium Pyruvate |
| Thymidine |

Examples of the biomarker include ornithine, 2-aminoadipic acid, deoxycytidine, glutamic acid, tryptophan, asparagine, alanine, cystine, hypoxanthine, uridine, aspartic acid, arginine, 2-hydroxybutyric acid, 2-hydroxyisovaleric acid, 3-hydroxyisovaleric acid, urea, 4-hydroxybenzoic acid, 4-aminobenzoic acid, ribonic acid, kynurenine, cystathionine, threonic acid, pyruvic acid, putrescine, ascorbic acid, riboflavin, serine, cysteine, orotic acid, and citric acid. In the present invention the biomarkers are selected from a group that consists of ornithine, 2-aminoadipic acid, deoxycytidine, tryptophan, hypoxanthine, uridine and kynurenine.

In a culture supernatant of cells in an undifferentiated state and in a culture supernatant of cells in a differentiated state, abundances of these biomarkers are different. Therefore, by measuring and comparing abundances of these biomarkers in a culture supernatant of the test cells and control cells, whether or not the cells are in a differentiated state or in an undifferentiated state can be determined. It is also possible to evaluate a differentiation state by measuring an abundance of a biomarker in a culture supernatant of control cells for which a differentiation state is known, and comparing an abundance of the indicator substance in a culture supernatant of test cells with the abundance of the indicator substance in the culture supernatant of the control cells.

The control cells are pluripotent stem cells. For differentiated cells, an abundance of a biomarker in a culture supernatant may vary due to a difference in a degree of progression of differentiation, a difference in directionality of differentiation, and the like. Therefore, pluripotent stem cells are used as control cells. Comparison of an abundance of a biomarker in a culture supernatant of test cells and an abundance of a biomarker in a culture supernatant of control cells is performed, for example, by obtaining a ratio of the two. When this ratio is greater than or equal to a predetermined threshold (the threshold is greater than 1) or less than or equal to a predetermined threshold (the threshold is less than 1), it can be determined that the cells are in a differentiated state. It is also possible that, when the ratio of the abundance of the biomarker in the culture supernatant of the test cells to the abundance of the biomarker in the culture supernatant of the control cells is the same as the predetermined threshold, it is determined that the cells are in an undifferentiated state, and, when the ratio exceeds the threshold or is less than the threshold, it is determined that the cells are in a differentiated states. When cells differentiation-induced from stem cells are the test cells, the term "undifferentiated state" may include a case where cells in an undifferentiated state are mixed in cells in a differentiated state.

In the cell differentiation state evaluation method according to the present invention, a differentiation state of test cells is evaluated by comparing abundances of two or more kinds of biomarkers in a culture supernatant of the test cells and control cells. By combining two or more kinds of biomarkers, the differentiation state can be evaluated with higher accuracy. Further, by combining two or more kinds of biomarkers, in addition to a differentiation state of cells, whether the cells in a differentiated state are differentiated into endoderm, mesoderm, or ectoderm cells, in other words, directionality of differentiation, can be evaluated.

Two or more kinds of biomarkers can be arbitrarily selected from the above biomarkers. Among the above biomarkers, examples of biomarkers useful for evaluating directionality of differentiation include kynurenine, 2-aminoadipic acid, ornithine, deoxycytidine, tryptophan, cystathionine, hypoxanthine, and uridine. Evaluation is performed based on abundances of two or more kinds of biomarkers selected from a group that consists of ornithine, 2-aminoadipic acid, deoxycytidine, tryptophan, hypoxanthine, uridine and kynurenine.

Examples of method for evaluating a differentiation state based on abundances of two or more kinds of biomarkers include: a method in which change with culture time of an abundance of each biomarker in a culture supernatant of test cells is analyzed; a method in which abundances of two or more kinds of biomarkers are subjected to multivariate analysis; a method in which ratios of abundances of two or more kinds of indicator substances are obtained; and the like. It is also possible to perform evaluation by combining these methods. Determination based on abundances of two kinds of biomarkers may be performed, for example, by plotting an abundance (or index value) of a first biomarker as a horizontal axis and an abundance (or index value) of a second biomarker as a vertical axis. When evaluation is performed based on abundances of three or more kinds of biomarkers, multivariate analysis is useful.

An example of two kinds of biomarkers is a combination of a metabolic product and its precursor in an anteroposterior relation of a metabolic pathway. By performing evaluation based on a ratio of a metabolic product to a precursor, evaluation accuracy of directionality of differentiation is improved. A precursor of a metabolic pathway is not limited to a metabolite with respect to a metabolic product, but may also be a substance of two or more before in the metabolic pathway. Examples of a combination of a precursor and a metabolic product include a combination of tryptophan and kynurenine, a combination of ornithine and putrescine, a combination of arginine and ornithine, and the like. A type of a metabolic pathway is not particularly limited, and combinations of a precursor and a metabolic product are not limited to the above combinations.

In a case where a ratio of a metabolic product to a precursor is obtained, evaluation may be performed based on a ratio of a metabolic product to a precursor in a culture supernatant of test cells, or a ratio of an abundance of a metabolic product to an abundance of a precursor in a culture supernatant of control cells may be compared with a ratio of an abundance of the metabolic product to an abundance of the precursor in a culture supernatant of the test cells. Further, it is also possible to perform evaluation by obtaining a ratio of an abundance of a metabolic product in a culture supernatant of control cells to an abundance of the metabolic product in a culture supernatant of test cells, and a ratio of an abundance of a precursor in a culture supernatant of the control cells to an abundance of the precursor in a culture supernatant of the test cells, and comparing these ratios.

As a method for measuring the abundances of the biomarkers in a culture supernatant, quantitative analysis using mass spectrometry, in particular, quantitative analysis using a liquid chromatography mass spectrometer (LC-MS) or a gas chromatography mass spectrometer (GC-MS) can be suitably used. However, the method for measuring the abundances of the biomarkers in a culture supernatant is not limited to this. For example, it is also possible that a sample obtained by subjecting a culture supernatant to a predetermined pretreatment is flowed together with an eluent into a column of a liquid chromatography (LC) apparatus, and components separated and eluted by the column are detected using a detector such as an ultraviolet-visible spectroscopic detector or an infrared spectroscopic detector, and, from results of the detection, abundances of the biomarkers contained in the sample are obtained. Further, it is also possible that a reagent or the like that specifically causes a biomarker to develop a color or to emit light is added to the culture supernatant, and an abundance of the biomarker is obtained based on an intensity of the color development or the light emission.

Example

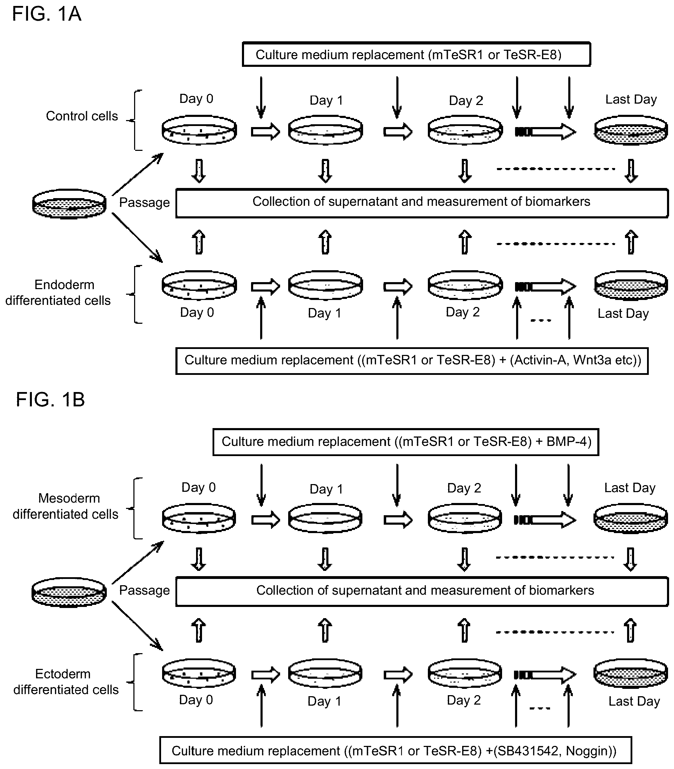

In the following, an example of evaluation of a differentiation state of cells using the method of the present invention is described. Figs. 1A and 1B are schematic diagrams illustrating procedures of a cell differentiation state evaluation method of the present example.

In the present example, human iPS cell lines PFX #9 were used. Further, cells imparting differentiation induction stimulation to the human iPS cell lines were used as test cells, and cells that do not impart differentiation induction stimulation to the human iPS cell lines (that is, cells that maintain an undifferentiated state) were used as control cells. In the following, procedures from cell culture to analysis of a culture supernatant in the present example are described.

### [Culture of Control Cells and Collection of Culture Supernatant]

Culture was performed by subculturing the PFX #9 lines in three culture dishes (diameter: 60 mm) coated with Vitronectin-N (Life Technologies Corporation) (in Fig. 1A, for simplicity, only one culture dish is illustrated). As a culture medium, mTeSR1 (modified Tenneille Serum Replacer 1, STEMCELL Technologies Corporation) or TeSR-E8 (Tenneille Serum Replacer E8, STEMCELL Technologies Corporation) was used, and culture medium replacement was performed daily. Components of mTeSR1 are shown in Table 2, and components of TeSR-E8 are shown in Table 3. The day when the subculture (passage) of the cells was performed was taken as Day 0, and the culture was continued for 6 days. A culture supernatant collected from a culture dish when the culture medium was replaced each day was used as a sample for mass spectrometry.

**[Table 2]**

| Components |
|---|
| DMEM/F12 |
| NaHCO3 |
| L-Ascorbic Acid |
| Selenium |
| Transferrin |
| Insulin |
| FGF2 |
| TGF-*β* |
| Bovine Serum Albumin(BSA) |
| Glutathione |
| Trace Elements |
| *β*-mercaptoethanol |
| Pipe colic acid |
| GABA |
| Lithium Chroride |
| Defined Lipids |

**[Table 3]**

| Components |
|---|
| DMEM/F12 |
| NaHCO3 |
| L-Ascorbic Acid |
| Selenium |
| Transferrin |
| Insulin |
| FGF2 |
| TGF-*β* |

### [Culture of Endoderm Differentiated Cells and Collection of Culture Supernatant]

Culture was performed by subculturing the PFX #9 lines in three culture dishes (diameter: 60 mm) coated with Vitronectin-N (in Fig. 1A, for simplicity, only one culture dish is illustrated). As a culture medium, mTeSR1 or TeSR-E8 was used, and culture medium replacement was performed daily, and culture was continued until a confluent state was reached. The day when the passage was performed was taken as Day 0. On Day 1, the culture medium was replaced with a culture medium obtained by adding Activin-A (PeproTech Corporation), Wnt3a (Wingless-type MMTV integration site family, member 3A, PeproTech Corporation), and BMP-4 (Bone Morphogenetic Proteins-4, PeproTech Corporation) such that final concentrations of Activin-A, Wnt3a, and BMP-4 were respectively 100 ng/mL, 40 ng/mL, and 0.5 ng/mL. On Day 2 and later, endoderm differentiation induction stimulation was performed by replacing the culture medium with a culture medium obtained by adding Activin-A and BMP-4 such that final concentrations of Activin-A and BMP-4 were respectively 100 ng/mL and 0.5 ng/mL. The day when the subculture (passage) of the cells was performed was taken as Day 0, and the culture was continued for 6 days. A culture supernatant collected from a culture dish when the culture medium was replaced each day was used as a sample for mass spectrometry.

### [Culture of Mesoderm Differentiated Cells and Collection of Culture Supernatant]

Culture was performed by subculturing the PFX #9 lines in three culture dishes (diameter: 60 mm) coated with Vitronectin-N (in Fig. 1B, for simplicity, only one culture dish is illustrated). As a culture medium, mTeSR1 or TeSR-E8 was used, and culture medium replacement was performed daily, and culture was continued until a confluent state was reached. The day when the passage was performed was taken as Day 0. On Day 1 and later, mesoderm differentiation induction stimulation was performed by replacing the culture medium with a culture medium obtained by adding BMP-4 such that a final concentration of BMP-4 was 40 ng/mL. The day when the subculture (passage) of the cells was performed was taken as Day 0, and the culture was continued for 6 days. A culture supernatant collected from a culture dish when the culture medium was replaced each day was used as a sample for mass spectrometry.

### [Culture of Ectoderm Differentiated Cells and Collection of Culture Supernatant]

Culture was performed by subculturing the PFX #9 lines in three culture dishes (diameter: 60 mm) coated with Vitronectin-N (in Fig. 1B, for simplicity, only one culture dish is illustrated). As a culture medium, mTeSR1 or TeSR-E8 was used, and culture medium replacement was performed daily, and culture was continued until a confluent state was reached. The day when the passage was performed was taken as Day 0. On Day 1 and later, ectoderm differentiation induction stimulation was performed by replacing the culture medium with a culture medium obtained by adding SB431542 (4-[4- (1,3-benzodioxol-5-yl)-S-(2-pyridinyl)-1H-imidazol-2-yl]benzamide, Wako Pure Chemical Industries, Ltd.) and Noggin (PeproTech Corporation) such that final concentrations of SB431542 and Noggin were respectively 10 µM and 500 ng/mL. The day when the subculture (passage) of the cells was performed was taken as Day 0, and the culture was continued for 6 days. A culture supernatant collected from a culture dish when the culture medium was replaced each day was used as a sample for mass spectrometry.

### [Analysis using LC-MS]

20 µL of 0.5 mM isopropyl malic acid aqueous solution as an internal standard substance was added to 100 µL of the above-described sample, and, after mixing, 200 µL of acetonitrile was added to remove protein. Thereafter, the sample was subjected to centrifugal separation (15,000 rpm, room temperature, 15 minutes), and a supernatant was collected, and was diluted 10-fold with ultrapure water (Milli-Q (registered trademark) water, Merck Corporation) and was then subjected to LC-MS analysis. The LC-MS analysis was performed according to analysis conditions recorded in "LC/MS/MS method package cell culture profiling" (hereinafter abbreviated as "MP") provided by Shimadzu Corporation. The MP is a collection of analysis condition parameters for analyzing, using LC-MS, compounds contained in the culture medium and metabolites secreted from the cells. Identification of a compound was performed based on that a difference between a retention time of a standard product registered in MP and a retention time of a compound in a sample was within ±0.3 minutes, that peaks of both quantification ions and confirmation ions were detected, and that an intensity value was 1000 or more. Further, quantification of a compound was performed using a method in which a mass chromatogram area for ions (quantification ions) characteristic to each compound in a sample was calculated.

### [Analysis using GC-MS]

10 µL of 0.5 mg/mL isopropyl malic acid aqueous solution as an internal standard substance was added to 100 µL of the above-described sample, and, after mixing, 200 µL of acetonitrile was added to remove protein. Thereafter, the sample was subjected to centrifugal separation (15,000 rpm, room temperature, 15 minutes), and 100 µL of a supernatant was collected, and drying under a reduced pressure was performed. By incubating each sample in a pyridine solution containing methoxyamine hydrochloride, methoximation of a compound in the sample was performed. Further, MSTFA (N-methyl-N-trimethylsilyl trifluoroacetamide) was added to each sample to trimethylsilylate (derivatize) a compound in the sample. Then, these samples subjected to derivatization treatment were subjected to analysis using GC-MS.

In the GC-MS analysis, "Smart Metabolites Database" (hereinafter abbreviated as "DB") provided by Shimadzu Corporation was used. The DB is a collection of data obtained by analyzing, using GC-MS, various standard products that have been subjected to the same treatment as the above-described derivatization treatment. Identification of a compound was performed using, as indicators, whether or not a difference between a retention index set in the DB (a relative value of a retention time) and a retention index of a derivatized compound in a sample was within ±5, and whether or not both quantification ions and confirmation ions set in the database were detected for a derivatized compound in the sample. On the other hand, quantification of a compound was performed using a method in which a mass chromatogram area for ions characteristic to each derivatized compound in a sample was calculated according to conditions set in the database.

### [Data Analysis]

With respect to each of culture supernatants collected from Day 1 to Day 6 of the culture, a value (area ratio) obtained by dividing a quantitative value (area value) of each compound obtained by the LC-MS analysis and the GC-MS analysis by a quantitative value (area value) of the internal standard substance was calculated, and this was taken as an index value of an abundance of the each compound in the culture supernatant. For each of the undifferentiated maintenance cells (control cells), the endoderm differentiated cells, the mesoderm differentiated cells, and the ectoderm differentiated cells, index values of abundances of an identified compound were plotted in a coordinate system in which a horizontal axis represents the number of culture days and a vertical axis represents an index value. Further, a compound for which a change in an index value of an abundance during the course of the culture was clearly different from that of the control cells was selected. Specifically, during a time period from Day 3 to Day 6 of the culture, when an average value of the index values of the control cells was A and an average value of the index values of the test cells was B, a compound for which A/B or B/A was 1.5 or more was selected.

Further, in order to select an effective compound for evaluating a state of cells at an initial stage of the culture, multivariate analysis was performed. Specifically, for the samples during a time period from Day 2 to Day 4 of the culture, the index values calculated above were input to multivariate analysis software (R version 3.1.2), and principal component analysis was performed. Based on the results of the analysis, a combination of compounds capable of distinguishing between the undifferentiated maintenance cells, the endoderm differentiated cells, the mesoderm differentiated cells, and the ectoderm differentiated cells was selected.

### [Results]

As a result, compounds for which a change in an index value of an abundance during the course of the culture in a culture supernatant of the control cells is clearly different from that in a culture supernatant of the test cells for which differentiation induction stimulation has been performed are shown in the following Tables 4 - 24. In these tables, a sample for which a background of a numerical value is shaded indicates a sample for which it is determined that there is a change relative to the index value of the control cells.

### [Table 4]

**Table 4 Kynurenine**

| | mTeSR1 | | | TeSR-E8 | | |
|---|---|---|---|---|---|---|
| | Endoderm Differentiation | Mesoderm Differentiation | Ectoderm Differentiation | Endoderm Differentiation | Mesoderm Differentiation | Ectoderm Differentiation |
| Day 3 | 0.18893 | 0.21818 | 0.73990 | 0.05575 | 0.11670 | 0.63681 |
| Day 4 | 0.12858 | 0.14785 | 0.59158 | 0.01649 | 0.03062 | 0.33124 |
| Day 5 | 0.19073 | 0.09843 | 0.46329 | 0.01260 | 0.01442 | 0.13219 |
| Dav 6 | 0.35260 | 0.03593 | 0.38332 | 0.01525 | 0.00786 | 0.06775 |

From Table 4, it is found that, for kynurenine, due to differentiation induction, an abundance in a culture supernatant of the test cells is decreased relative to an abundance in a culture supernatant of the control cells. Therefore, in a case where undifferentiated cells are used as the control cells, when a ratio ((test cells)/(control cells)) of an abundance in a culture supernatant of the test cells to an abundance of kynurenine in a culture supernatant of the control cells is equal to or less than a predetermined threshold, it can be determined that the test cells are in a differentiated state.

### [Table 5]

**Table 5 Ornithine**

| | mTeSR1 | | | TeSR-E8 | | |
|---|---|---|---|---|---|---|
| | Endoderm Differentiation | Mesoderm Differentiation | Ectoderm Differentiation | Endoderm Differentiation | Mesoderm Differentiation | Ectoderm Differentiation |
| Day 3 | 0.85153 | 0.61255 | 0.92215 | 0.47613 | 0.40499 | 1.04040 |
| Day 4 | 0.82127 | 0.58431 | 0.96502 | 0.35876 | 0.16643 | 0.93894 |
| Day 5 | 0.68689 | 0.59170 | 0.88162 | 0.35903 | 0.12975 | 0.90758 |
| Day 6 | 0.67821 | 0.49754 | 0.77119 | 0.30423 | 0.14435 | 0.91217 |

From Table 5, it is found that, for ornithine, due to differentiation induction, an abundance in a supernatant of the test cells is decreased as compared to the control cells. Therefore, in the case where undifferentiated cells are used as the control cells, when a ratio ((test cells)/(control cells)) of an abundance in a culture supernatant of the test cells to an abundance of ornithine in a culture supernatant of the control cells is equal to or less than a predetermined threshold, it can be determined that the test cells are in a differentiated state.

### [Table 6]

**Table6 2-Aminoadipic acid**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 0.05193 | 0.58617 | 1.80826 | 0.26223 | 0.34349 | 7.45760 |
| Day 4 | 0.02984 | 0.47606 | 2.11081 | 0.20057 | 0.20629 | 7.26711 |
| Day 5 | 0.03172 | 0.50292 | 2.40063 | 0.18995 | 0.22813 | 6.35465 |
| Day 6 | 0.07205 | 0.70721 | 3.13504 | 0.18617 | 0.10794 | 3.68514 |

From Table 6, it is found that, for 2-aminoadipic acid, for the test cells differentiation-induced into endoderm and mesoderm, an abundance in a supernatant is decreased as compared to the control cells. On the other hand, it is found that, for the test cells differentiation-induced into ectoderm, an abundance in a supernatant is increased as compared to the control cells for all Day 3 - Day 6 of the culture. Therefore, in the case where undifferentiated cells are used as the control cells, when a ratio ((test cells)/(control cells)) of an abundance in a culture supernatant of the test cells to an abundance of 2-aminoadipic acid in a culture supernatant of the control cells is equal to or less than a predetermined threshold, it can be determined that the test cells are differentiated toward endoderm or mesoderm, and when the ratio is equal to or greater than the threshold, it can be determined that the test cells are differentiated toward ectoderm.

### [Table 7]

**Table7 Deoxycytidine**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 0.12568 | 1.67146 | 0.97320 | 0.27835 | 0.95542 | 0.38476 |
| Day 4 | 0.05936 | 2.03705 | 1.32781 | 0.11147 | 1.62561 | 0.91033 |
| Day 5 | 0.07383 | 3.15055 | 1.35494 | 0.25834 | 4.85835 | 3.23319 |
| Day 6 | 0.11886 | 3.18575 | 0.84337 | 0.24061 | 4.60401 | 2.07900 |

From Table 7, it is found that, for deoxycytidine, for the test cells differentiation-induced into endoderm, an abundance in a supernatant is decreased as compared to the control cells. On the other hand, it is found that, for the test cells differentiation-induced into mesoderm and some of the test cells differentiation-induced into ectoderm, an abundance in a supernatant is increased as compared to the control cells. Therefore, in the case where undifferentiated cells are used as the control cells, when a ratio ((test cells)/(control cells)) of an abundance in a culture supernatant of the test cells to an abundance of deoxycytidine in a culture supernatant of the control cells is equal to or less than a predetermined threshold, it can be determined that the test cells are differentiated toward endoderm, and when the ratio is equal to or greater than the threshold, it can be determined that the test cells are differentiated toward mesoderm or ectoderm.

### [Table 8]

**Table8 Glutamicacid**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 1.04830 | 1.03500 | 0.91965 | 1.17905 | 1.03364 | 0.98334 |
| Day 4 | 1.25414 | 1.02633 | 0.98379 | 1.66323 | 0.95813 | 1.04074 |
| Day 5 | 1.64594 | 0.92395 | 1.24662 | 1.91679 | 0.75864 | 1.03077 |
| Day 6 | 1.46709 | 0.80920 | 0.87426 | 1.53753 | 1.07436 | 1.02387 |

From Table 8, it is found that, for glutamic acid, an abundance in a supernatant of the test cells differentiation-induced into endoderm is increased as compared to the control cells. Therefore, in the case where undifferentiated cells are used as the control cells, when a ratio ((test cells)/(control cells)) of an abundance in a culture supernatant of the test cells to an abundance of glutamic acid in a culture supernatant of the control cells is equal to or greater than a predetermined threshold, it can be determined that the test cells are differentiated toward endoderm.

### [Table 9]

**Table 9 Tryptophan**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 1.09811 | 1.21260 | 1.10550 | 1.02888 | 1.03623 | 1.03966 |
| Day 4 | 2.70456 | 3.29632 | 2.55021 | 1.26312 | 1.34472 | 1.30520 |
| Day 5 | 61.95333 | 85.32587 | 49.71053 | 2.44475 | 2.25226 | 2.26845 |
| Day 6 | 50.22615 | 105.71030 | 39.13180 | 3.10929 | 3.15314 | 3.11552 |

From Table 9, it is found that, for tryptophan, due to differentiation induction, an abundance in a supernatant of the test cells is increased as compared to an abundance in a supernatant of the control cells. Therefore, in the case where undifferentiated cells are used as the control cells, when a ratio ((test cells)/(control cells)) of an abundance in a culture supernatant of the test cells to an abundance of tryptophan in a culture supernatant of the control cells is equal to or greater than a predetermined threshold, it can be determined that the test cells are in a differentiated state.

### [Table 10]

**Table 10 Cystathionine**

| | mTe5R1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 1.16223 | 0.82639 | 0.76162 | 0.91514 | 0.91300 | 0.92984 |
| Day 4 | 1.02042 | 0.53625 | 0.76685 | 0.67659 | 0.48016 | 0.75582 |
| Day 5 | 0.77199 | 0.21684 | 0.74798 | 0.46001 | 0.38621 | 0.68651 |
| Day 6 | 0.78068 | 0.22021 | 0.77175 | 0.39274 | 0.39633 | 0.84683 |

From Table 10, it is found that, for cystathionine, due to differentiation induction, an abundance in a supernatant of the test cells is generally decreased as compared to an abundance in a supernatant of the control cells. Therefore, in the case where undifferentiated cells are used as the control cells, when a ratio ((test cells)/(control cells)) of an abundance in a culture supernatant of the test cells to an abundance of cystathionine in a culture supernatant of the control cells is equal to or less than a predetermined threshold, it can be determined that the test cells are in a differentiated state.

### [Table 11]

**Table 11 Alanine**

| | mTeSR1 | | | TeSR-E8 | | |
|---|---|---|---|---|---|---|
| Endoderm Differentiation Mesoderm | | Differentiation | Ectoderm Differentiation | Endoderm Differentiation | Differentiation | Ectoderm Differentiation |
| Day 3 | 0.93195 | 0.80433 | 0.94320 | 0.96237 | 0.77472 | 1.05763 |
| Day 4 | 1.01651 | 0.83640 | 0.92764 | 0.96875 | 0.71357 | 0.97390 |
| Day 5 | 0.85849 | 0.78237 | 0.84835 | 0.78790 | 0.62014 | 0.73593 |
| Dav 6 | 0.73327 | 0.61276 | 0.75377 | 0.59205 | 0.56090 | 0.72836 |

From Table 11, it is found that, for alanine, due to differentiation induction, an abundance in a supernatant of the test cells is generally decreased as compared to an abundance in a supernatant of the control cells. Therefore, in the case where undifferentiated cells are used as the control cells, when a ratio ((test cells)/(control cells)) of an abundance in a culture supernatant of the test cells to an abundance of alanine in a culture supernatant of the control cells is equal to or less than a predetermined threshold, it can be determined that the test cells are in a differentiated state.

### [Table 12]

**Table 12 Cystine**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 0.91086 | 1.06517 | 1.09896 | 0.87903 | 0.92513 | 0.91374 |
| Day 4 | 1.14269 | 1.94349 | 1.11833 | 0.69469 | 1.13958 | 0.92408 |
| Day 5 | 0.79866 | 2.28473 | 0.82651 | 0.47528 | 1.48378 | 0.86547 |
| Day 6 | 0.89009 | 3.74203 | 1.01226 | 0.47205 | 1.37228 | 0.61081 |

From Table 12, it is found that, for cystine, for the test cells differentiation-induced into endoderm and ectoderm, an abundance in a supernatant is generally decreased as compared to the control cells. On the other hand, it is found that, for the test cells differentiation-induced into mesoderm, an abundance in a supernatant is generally increased as compared to the control cells. Therefore, in the case where undifferentiated cells are used as the control cells, when a ratio ((test cells)/(control cells)) of an abundance in a culture supernatant of the test cells to an abundance of cystine in a culture supernatant of the control cells is equal to or less than a predetermined threshold, it can be determined that the test cells are differentiated toward endoderm or ectoderm, and when the ratio is equal to or greater than the threshold, it can be determined that the test cells are differentiated toward mesoderm.

### [Table 13]

**Table 13 Hypoxanthine**

| | mTe5R1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 0.48051 | 2.98594 | 6.57292 | 0.47858 | 1.47942 | 4.55676 |
| Day 4 | 0.90741 | 3.46975 | 4.66758 | 0.98274 | 5.88716 | 50.41902 |
| Day 5 | 3.76607 | 10.33399 | 0.95857 | 3.32066 | 3.95113 | 40.10208 |
| Day 6 | 2.02114 | 10.69372 | 1.78502 | 0.42838 | 0.58486 | 3.44223 |

From Table 13, it is found that, for hypoxanthine, for the test cells differentiation-induced into mesoderm and ectoderm, an abundance in a supernatant is generally increased as compared to the control cells until Day 5 of the culture. Therefore, for hypoxanthine, in the case where undifferentiated cells are used as the control cells, during a time period from the start to Day 5 of the culture, when a ratio ((test cells)/(control cells)) of an abundance in a culture supernatant of the test cells to an abundance of hypoxanthine in a culture supernatant of the control cells is equal to or greater than a predetermined threshold, it can be determined that the test cells are differentiated toward mesoderm or ectoderm.

### [Table 14]

**Table 14 Uridine**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 1.27119 | 1.06620 | 1.16740 | 0.95583 | 1.05244 | 0.92484 |
| Day 4 | 1.92446 | 1.72021 | 1.14304 | 2.73917 | 2.51786 | 0.97607 |
| Day 5 | 4.60898 | 3.82077 | 0.83762 | 5.06128 | 1.83698 | 1.53363 |
| Day 6 | 1.33203 | 1.72377 | 0.86755 | 3.26169 | 1.17087 | 1.23247 |

From Table 14, it is found that, for uridine, due to differentiation induction, an abundance in a supernatant of the test cells is increased as compared to an abundance in a supernatant of the control cells. Therefore, in the case where undifferentiated cells are used as the control cells, when a ratio ((test cells)/(control cells)) of an abundance in a culture supernatant of the test cells to an abundance of uridine in a culture supernatant of the control cells is equal to or greater than a predetermined threshold, it can be determined that the test cells are in a differentiated state.

### [Table 15]

**Table 15 Aspartic acid**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 0.95932 | 0.96292 | 0.94153 | 1.08864 | 1.00146 | 1.01118 |
| Day 4 | 0.99362 | 0.94460 | 0.90981 | 1.16674 | 0.84659 | 0.92238 |
| Day 5 | 1.07273 | 1.01921 | 1.02430 | 1.21080 | 0.63493 | 0.80039 |
| Dav 6 | 0.87298 | 0.92329 | 0.66322 | 0.98378 | 0.59203 | 0.75007 |

From Table 15, it is found that, for aspartic acid, due to differentiation induction, an abundance in a supernatant of the test cells is decreased as compared to an abundance in a supernatant of the control cells. Therefore, in the case where undifferentiated cells are used as the control cells, when a ratio ((test cells)/(control cells)) of an abundance in a culture supernatant of the test cells to an abundance of aspartic acid in a culture supernatant of the control cells is equal to or less than a predetermined threshold, it can be determined that the test cells are in a differentiated state.

### [Table 16]

**Table 16 Arginine**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Dav 3 | 0.94176 | 1.04160 | 0.87727 | 1.04290 | 1.05220 | 0.98153 |
| Day 4 | 1.18873 | 1.37727 | 0.97998 | 1.16855 | 1.20608 | 0.96800 |
| Day 5 | 1.72819 | 1.89957 | 1.27474 | 1.42427 | 1.42390 | 1.07959 |
| Day 6 | 2.21368 | 2.85952 | 1.56629 | 1.41294 | 1.51619 | 1.03256 |

From Table 16, it is found that, for arginine, due to differentiation induction, an abundance in a supernatant of the test cells is generally increased as compared to an abundance in a supernatant of the control cells. Therefore, in the case where undifferentiated cells are used as the control cells, when a ratio ((test cells)/(control cells)) of an abundance in a culture supernatant of the test cells to an abundance of arginine in a culture supernatant of the control cells is equal to or greater than a predetermined threshold, it can be determined that the test cells are in a differentiated state.

### [Table 17]

**Table 17 2-Hydroxybutyric acid**

| | mTe5R1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 1.07430 | 0.95423 | 0.78712 | 0.97805 | 0.96107 | 1.06073 |
| Day 4 | 0.92993 | 0.84393 | 0.62321 | 0.96395 | 1.01651 | 0.89628 |
| Day 5 | 0.48145 | 0.86498 | 0.69611 | 0.75546 | 0.73369 | 0.73792 |
| Day 6 | 0.47994 | 0.46706 | 0.44935 | 0.54717 | 0.43526 | 0.59593 |

From Table 17, it is found that, for 2-hydroxybutyric acid, due to differentiation induction, an abundance in a supernatant of the test cells is decreased as compared to an abundance in a supernatant of the control cells. Therefore, in the case where undifferentiated cells are used as the control cells, when a ratio ((test cells)/(control cells)) of an abundance in a culture supernatant of the test cells to an abundance of 2-hydroxybutyric acid in a culture supernatant of the control cells is equal to or less than a predetermined threshold, it can be determined that the test cells are in a differentiated state.

### [Table 18]

**Table 18 2-Hydroxyisovaleric acid**

| | mTeXSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 1.27255 | 0.98944 | 1.10056 | 1.30692 | 1.38960 | 1.42050 |
| Day 4 | 1.79894 | 0.75779 | 1.24476 | 1.35047 | 0.83504 | 1.01369 |
| Day 5 | 0.61928 | 0.29113 | 0.41913 | 0.92482 | 0.53223 | 0.64369 |
| Dav 6 | 0.92244 | 0.46552 | 0.69112 | 0.71194 | 0.62099 | 0.73549 |

From Table 18, it is found that, for 2-hydroxyisovaleric acid, due to differentiation induction, an abundance in a supernatant of the test cells is generally decreased as compared to the control cells. Therefore, in the case where undifferentiated cells are used as the control cells, when a ratio ((test cells)/(control cells)) of an abundance in a culture supernatant of the test cells to an abundance of 2-hydroxyisovaleric acid in a culture supernatant of the control cells is equal to or less than a predetermined threshold, it can be determined that the test cells are in a differentiated state.

### [Table 19]

**Table 19 3-Hydroxyisovaleric acid**

| | mTeSR1 | | | | TeSR-E8 | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 1.02519 | 0.85818 | 1.15222 | 1.02921 | 1.18842 | 0.88045 |
| Dav 4 | 0.65141 | 1.03118 | 1.13604 | 0.37724 | 0.72505 | 0.37461 |
| Day 5 | 0.71332 | 1.13614 | 1.43775 | 0.27049 | 0.27218 | 0.30534 |
| Dav 6 | 1.03230 | 0.60868 | 1.20830 | 0.38348 | 0.17757 | 0.36216 |

From Table 19, it is found that, for 3-hydroxyisovaleric acid, due to differentiation induction, except for some of the test cells differentiation-induced into endoderm and ectoderm, an abundance in a supernatant of the test cells is decreased as compared to an abundance in a supernatant of the control cells. Therefore, in the case where undifferentiated cells are used as the control cells, when a ratio ((test cells)/(control cells)) of an abundance in a culture supernatant of the test cells to an abundance of 3-hydroxyisovaleric acid in a culture supernatant of the control cells is equal to or less than a predetermined threshold, it can be determined that the test cells are in a differentiated state.

### [Table 20]

**Table 20 Urea**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 0.92310 | 0.70312 | 1.01352 | 0.64806 | 0.66719 | 1.27356 |
| Dav 4 | 0.80755 | 0.71764 | 1.07742 | 0.41185 | 0.21851 | 1.00317 |
| Day 5 | 0.65412 | 0.61466 | 0.96093 | 0.38509 | 0.20195 | 0.92338 |
| Dav 6 | 0.63476 | 0.50105 | 0.79971 | 0.30602 | 0.12709 | 0.85022 |

From Table 20, it is found that, for urea, due to differentiation induction, except for some of the test cells differentiation-induced into ectoderm, an abundance in a supernatant of the test cells is decreased as compared to an abundance in a supernatant of the control cells. In the case where undifferentiated cells are used as the control cells, when a ratio ((test cells)/(control cells)) of an abundance in a culture supernatant of the test cells to an abundance of urea in a culture supernatant of the control cells is equal to or less than a predetermined threshold, it can be determined that the test cells are in a differentiated state.

### [Table 21]

**Table 21 4-Hydroxybenzoic acid**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Dav 3 | 1.27722 | 0.67413 | 0.88865 | 1.64980 | 0.76347 | 1.10956 |
| Dav 4 | 1.11066 | 0.53828 | 0.71428 | 0.67843 | 0.44439 | 0.73755 |
| Day 5 | 0.63066 | 0.51684 | 0.53872 | 0.33834 | 0.37585 | 0.39470 |
| Day 6 | 0.72489 | 0.41491 | 0.67327 | 0.32236 | 0.30864 | 0.31271 |

From Table 21, it is found that, for 4-hydroxybenzoic acid, due to differentiation induction, except for some of the test cells differentiation-induced into endoderm and some of the test cells differentiation-induced into ectoderm, an abundance in a supernatant of the test cells is decreased as compared to an abundance in a supernatant of the control cells. Therefore, in the case where undifferentiated cells are used as the control cells, when a ratio ((test cells)/(control cells)) of an abundance in a culture supernatant of the test cells to an abundance of 4-hydroxybenzoic acid in a culture supernatant of the control cells is equal to or less than a predetermined threshold, it can be determined that the test cells are in a differentiated state.

### [Table 22]

**Table 22 4-Aminobenzoic acid**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Day 3 | 1.24586 | 1.49127 | 3.01381 | 1.29965 | 1.04855 | 2.81920 |
| Day 4 | 1.29134 | 1.31592 | 3.01235 | 1.15175 | 1.11488 | 2.41449 |
| Day 5 | 2.61237 | 1.08518 | 1.17458 | 1.16881 | 1.76581 | 3.06917 |
| Day 6 | 1.13642 | 1.05606 | 2.41449 | 1.10461 | 1.09812 | 2.07817 |

From Table 22, it is found that, for 4-aminobenzoic acid, due to differentiation induction, an abundance in a supernatant of the test cells is increased as compared to an abundance in a supernatant of the control cells. Therefore, in the case where undifferentiated cells are used as the control cells, when a ratio ((test cells)/(control cells)) of an abundance in a culture supernatant of the test cells to an abundance of 4-aminobenzoic acid in a culture supernatant of the control cells is equal to or greater than a predetermined threshold, it can be determined that the test cells are in a differentiated state.

### [Table 23]

**Table 23 Ribonic acid**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation | Ectoderm Differentiation |
| Dav 3 | 1.06590 | 0.84094 | 1.03030 | 1.08877 | 0.73380 | 1.37594 |
| Dav 4 | 1.05338 | 0.68870 | 0.97971 | 1.15439 | 0.48453 | 1.11639 |
| Day 5 | 1.04836 | 0.46572 | 1.03581 | 1.14568 | 0.31384 | 1.14763 |
| Dav 6 | 0.96847 | 0.34366 | 0.90276 | 1.11614 | 0.35749 | 1.11192 |

From Table 23, it is found that, for ribonic acid, for the test cells differentiation-induced into mesoderm, an abundance in a supernatant is decreased as compared to the control cells. Therefore, in the case where undifferentiated cells are used as the control cells, when a ratio ((test cells)/(control cells)) of an abundance in a culture supernatant of the test cells to an abundance of ribonic acid in a culture supernatant of the control cells is equal to or less than a predetermined threshold, it can be determined that the test cells are differentiated toward mesoderm.

### [Table 24]

**Table 24 Citric acid**

| | mTeSR1 | | | TeSR-E8 | | |
|---|---|---|---|---|---|---|
| Endoderm Differentiation | | Mesoderm Differentiation | Ectoderm Differentiation | Endoderm Differentiation | Mesoderm Differentiation Ectoderm | Differentiation |
| Day 3 | 1.06624 | 0.98091 | 0.92678 | 7.21362 | 1.38785 | 1.23408 |
| Day 4 | 1.10012 | 1.59673 | 0.85179 | 5.09693 | 2.15716 | 0.84740 |
| Day 5 | 0.91417 | 2.12469 | 0.93419 | 3.38313 | 3.13229 | 0.58426 |
| Dav 6 | 1.00922 | 2.18626 | 1.22431 | 2.23508 | 4.18140 | 0.49242 |

From Table 24, it is found that, for citric acid, for the test cells differentiation-induced into mesoderm and some of the test cells differentiation-induced into endoderm, due to differentiation induction, an abundance in a supernatant of the test cells is increased as compared to the control cells. Therefore, in the case where undifferentiated cells are used as the control cells, when a ratio ((test cells)/(control cells)) of an abundance in a culture supernatant of the test cells to an abundance of citric acid in a culture supernatant of the control cells is equal to or less than a predetermined threshold, it can be determined that the test cells are differentiated toward endoderm or mesoderm.

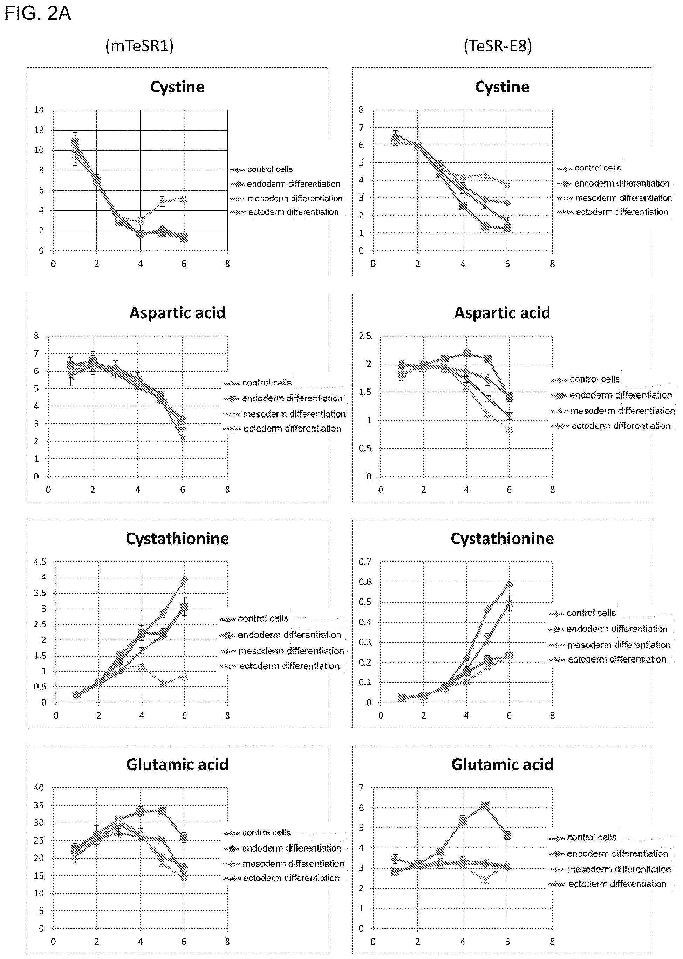

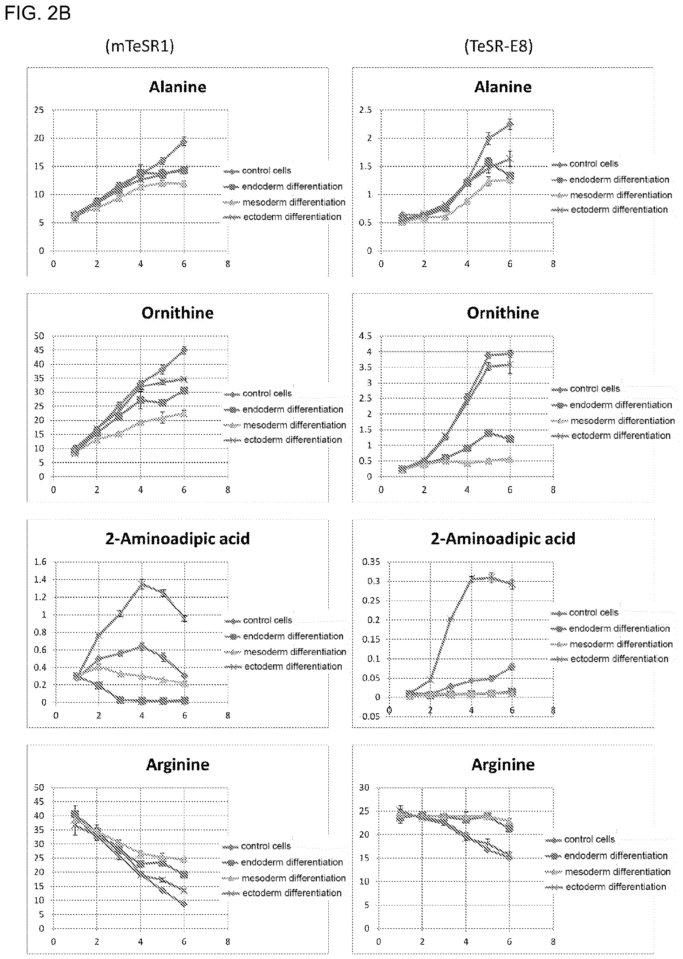

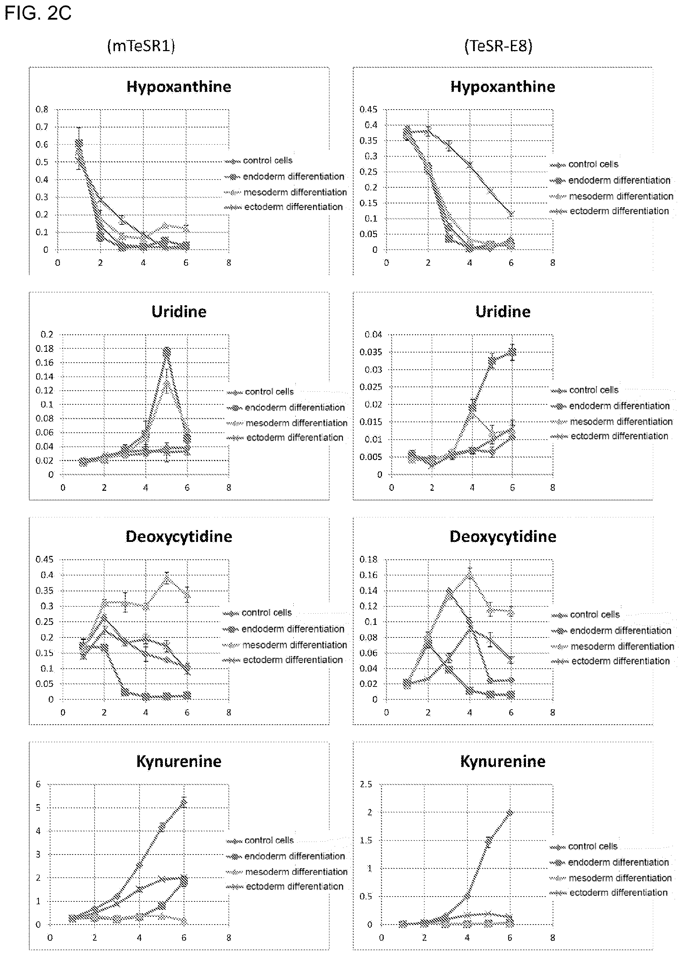

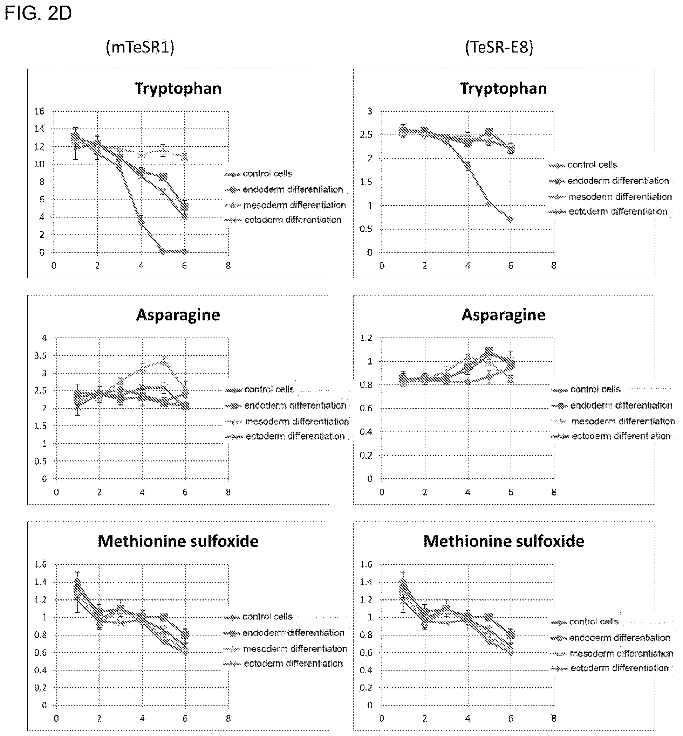

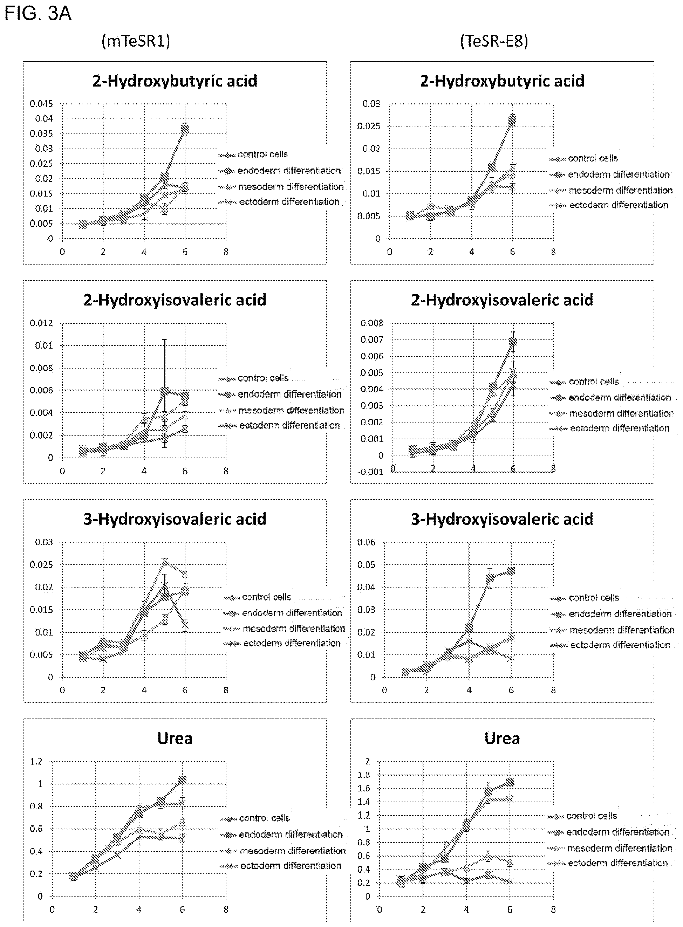

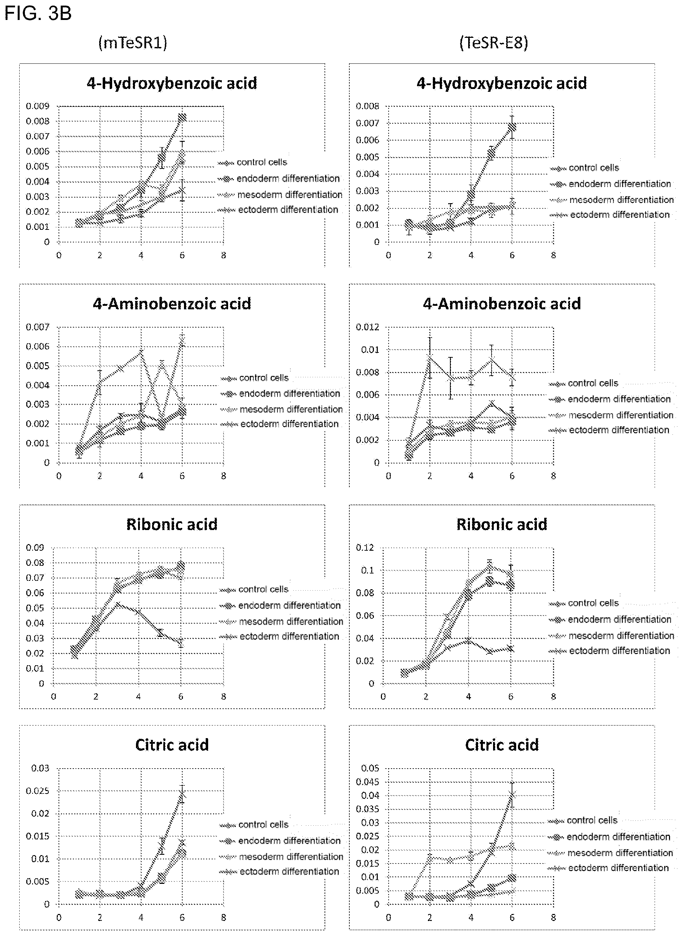

Figs. 2A, 2B, 2C, 2D, 3A and 3B show changes in abundance of the biomarkers in culture supernatants of Day 1 - Day 6 of culture of mTeSR1 lines and TeSR-E8 lines. Figs. 2A - 2D show results of LC-MS analyses, and Figs. 3A and 3B show results of GC-MS analyses. As is clear from these figures, it is confirmed that, although there are no differences in the abundances of the biomarkers between the test cells and the control cells, for many of the biomarkers, the differences in the abundances between the test cells and the control cells increase as time passes. It is confirmed that the time when the difference in abundance between the control cells and the test cells becomes the largest depends on the kind of the cultured cells and the kind of the biomarker. Further, it is also confirmed that there are differences in abundances of biomarkers depending on the differentiation state of the test cells (the endoderm differentiated cells, the mesoderm differentiated cells, and the ectoderm differentiated cells).

For example, for kynurenine, relative to the test cells, an abundance in a culture supernatant of the control cells greatly increases as culture time passes. For 2-aminoadipic acid, although there are nearly no changes in abundances in culture supernatants of the endoderm differentiated cells and the mesoderm differentiated cells, there are changes (increases) in abundances in culture supernatants of the control cells and the ectoderm differentiated cells, and in particular, there is a large change for the ectoderm differentiated cells. For deoxycytidine, after seeding, there are changes (increases) in abundances in culture supernatants of all the cells. However, for the cells subjected to differentiation induction stimulation to endoderm, an abundance in a culture supernatant greatly decreases as culture time passes. Therefore, by utilizing such a feature of each biomarker regarding a change in an abundance in a culture supernatant, it is possible to determine whether or not the test cells are in a differentiated state or in an undifferentiated state, and to evaluate the differentiation directionality (endoderm, mesoderm, or ectoderm) of the test cells that have been determined as being in a differentiated state. In evaluation based on only one kind of a biomarker, it is difficult to evaluate all of endoderm differentiation, mesoderm differentiation and ectoderm differentiation. However, when determination is performed based on abundances of two or more kinds of biomarkers, the differentiation directionality can be more accurately evaluated.

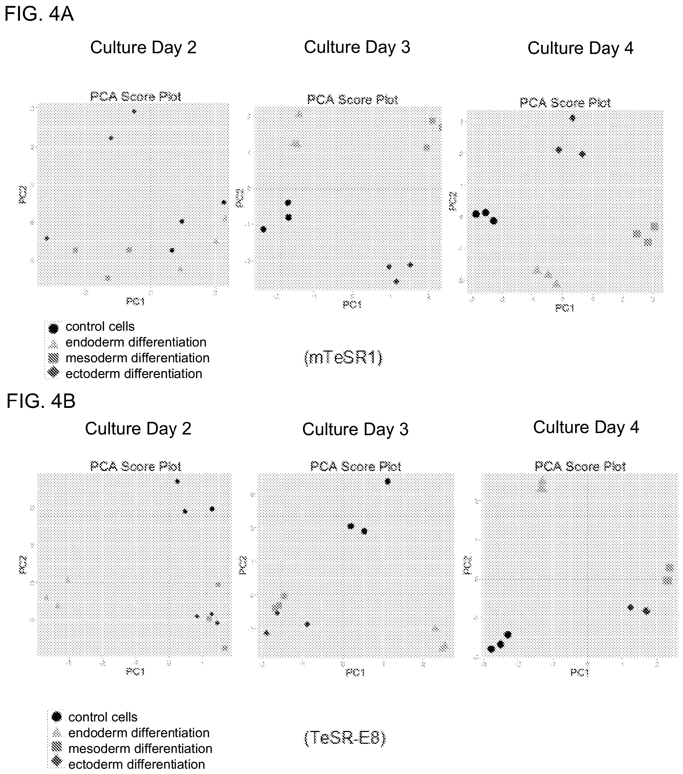

Fig. 4A shows results of principal component analyses of abundances of kynurenine, 2-aminoadipic acid, ornithine, deoxycytidine, tryptophan, cystathionine, hypoxanthine, and uridine in culture supernatants of Day 2 - Day 4 of culture of mTeSR1 lines. From this figure, it is found that, on Day 3 of the culture, the undifferentiated state (the control cells) can be clearly distinguished from each of the endoderm differentiated cells, the mesoderm differentiated cells and the ectoderm differentiated cells.

Further, Fig. 4B shows results of principal component analysis of abundances of the biomarkers in culture supernatants of Day 2 - Day 4 of culture of TeSR-E8 lines. From this figure, it is found that, on Day 4 of the culture, the undifferentiated state (the control cells) can be clearly distinguished from each of the endoderm differentiated cells, the mesoderm differentiated cells and the ectoderm differentiated cells.

That is, as shown in Figs. 4A and 4B, it is found that, by using abundances of two or more kinds of biomarkers in a culture supernatant as indicators, it is possible to determine whether or not the test cells are in a differentiated state or in an undifferentiated state, and to evaluate differentiation directionality (endoderm, mesoderm, or ectoderm) of the test cells.

### [Comparison with Other Test Methods]

Analysis based on cell destructive inspection was performed in order to confirm consistency between analysis of an abundance of a biomarker in a culture supernatant and analysis based on other test methods.

Similar to "the culture of the control cells," "the culture of the endoderm differentiated cells," "the culture of the mesoderm differentiated cells" and "the culture of the ectoderm differentiated cells" described above, mTeSR1 or TeSR-E8 was used as a culture medium, culture of cells and differentiation induction stimulation were performed, and the day when passage was performed was taken as Day 0, and cells were collected after Day 4 and after Day 7.
mRNA of the collected cells was extracted using TaqMan HPSC Scorecard Panel (Applied Biosystems Corporation), and 96 kinds of gene expression levels were evaluated using quantitative PCR, and, based on the results, scores of degrees of differentiation into the germ layers were calculated. Analysis scores of the mTeSR1 lines are shown in Table 25, and analysis scores of the TeSR-E8 lines are shown in Table 26. In these tables, for a sample for which a background of a numerical value is shaded, a analysis score is 0.5 or more, and it indicates that the cells are differentiated in a specific direction.

**[Table 25]**

| mTeSR1 | | Analysis Score | |
|---|---|---|---|
| | Endoderm Differentiation | Mesoderm Differentiation | Ectoderm Differentiation |
| Control Cells Day 4 | -1.70 | -1.06 | -1.43 |
| Control Cells Day 7 | -2.07 | -1.44 | -1.39 |
| Endoderm Differentiation Day 4 | 0.60 | -0.54 | -1.30 |
| Endoderm Differentiation Day 7 | 0.75 | -0.51 | -0.86 |
| Mesoderm Differentiation Day 4 | -1.57 | 0.50 | -1.41 |
| Mesoderm Differentiation Day 7 | -1.71 | 3.03 | -0.41 |
| Ectoderm Differentiation Day 4 | -1.68 | -0.90 | -0.29 |
| Ectoderm Differentiation Day 7 | -1.24 | -0.63 | 1.60 |

**[Table 26]**

| TeSR-E8 | | Analysis Score | |
|---|---|---|---|
| | Endoderm Differentiation | Mesoderm Differentiation | Ectoderm Differentiation |
| Control Cells Day 4 | -1.95 | -1.36 | -1.49 |
| Control Cells Day 7 | -1.53 | -0.72 | -1.28 |
| Endoderm Differentiation Day 4 | 0.24 | 0.26 | -1.43 |
| Endoderm Differentiation Day 7 | 1.02 | 0.76 | -0.57 |
| Mesoderm Differentiation Day 4 | -1.25 | 1.59 | -1.12 |
| Mesoderm Differentiation Day 7 | -0.64 | 4.58 | 0.00 |
| Ectoderm Differentiation Day 4 | -1.50 | -1.58 | -0.57 |
| Ectoderm Differentiation Day 7 | -1.21 | -0.11 | 0.64 |

From the results of Tables 25 and 26, it is found that, in "the culture of the control cells" (no differentiation induction stimulation), differentiation toward a specific direction is not observed; in "the culture of the endoderm differentiated cells" (differentiation induction stimulation by Activin-A, Wnt3a and BMP-4), endoderm differentiation proceeds; in "the culture of the mesoderm differentiated cells" (differentiation induction stimulation by BMP-4), mesoderm differentiation proceeds; and in "the culture of the ectoderm differentiated cells" (differentiation induction stimulation by B431542 and Noggin), ectoderm differentiation proceeds.

From these results, it can be said that the evaluation method in which an abundance of a biomarker in a culture supernatant is used as an indicator can reproduce the conventional cell destructive method, and is an excellent method in which change with time of a differentiation state can be evaluated with cells in the same culture dish as targets.

### [Data Analysis based on Ratio of Compounds in Anteroposterior Relation of Metabolic Pathway]

For culture supernatants collected from Day 1 to Day 6 of the culture, index values of abundances of substances obtained by LC-MS analysis were calculated. From the index values of kynurenine, tryptophan, ornithine, arginine and putrescine in the culture supernatants of the control cells (undifferentiated maintenance cells), the test cells (endoderm differentiated cells, mesoderm differentiated cells, and ectoderm differentiated cells), a value B/A was calculated which was obtained by dividing an average value B of the index values of the test cells by an average value A of the index values of the control cells.

### (Kynurenine/Tryptophan)

Tables 27 and 28 show values of B/A of kynurenine as a metabolic product, and values obtained by dividing B/A of kynurenine by B/A of tryptophan which is a precursor of the kynurenine pathway.

### [Table 27]

**Table 27 Kynurenine/Tryptophan (mTeSR1)**

| | mTeSR1 | | | | | |
|---|---|---|---|---|---|---|
| | Endoderm Differentiation | | Mesoderm Differentiation | | Ectoderm Differentiation | |
| | Kynurenine Alone | Kynurenine /Tryptophan | Kynurenine Alone | Kynurenine /Tryptophan | Kynurenine Alone | Kynurenine /Tryptophan |
| Day 1 | 0.9023 | 0.9163 | 0.9199 | 0.9661 | 0.8529 | 0.9634 |
| Day 2 | 0.4486 | 0.4105 | 0.5372 | 0.5070 | 0.7379 | 0.6674 |
| Day 3 | 0.1889 | 0.1721 | 0.2182 | 0.1799 | 0.7399 | 0.6693 |
| Day 4 | 0.1286 | 0.0475 | 0.1479 | 0.0449 | 0.5916 | 0.2320 |
| Day 5 | 0.1907 | 0.0031 | 0.0984 | 0.0012 | 0.4633 | 0.0093 |
| Day 6 | 0.3526 | 0.0070 | 0.0359 | 0.0003 | 0.3833 | 0.0098 |

### [Table 28]

**Table 28 Kynurenine/Tryptophan (TeSR-E8)**

| | TeSR-E8 | | | | | |
|---|---|---|---|---|---|---|
| | Endoderm Differentiation | | Mesoderm Differentiation | | Ectoderm Differentiation | |
| | Kynurenine Alone | Kynurenine /Tryptophan | Kynurenine Alone | Kynurenine /Tryptophan | Kynurenine Alone | Kynurenine /Tryptophan |
| Day 1 | 0.9681 | 0.9769 | 0.9745 | 0.9910 | 1.1081 | 1.1096 |
| Day 2 | 0.5220 | 0.5055 | 0.4253 | 0.4203 | 0.6351 | 0.6158 |
| Day 3 | 0.0557 | 0.0542 | 0.1167 | 0.1126 | 0.6368 | 0.6125 |
| Day 4 | 0.0165 | 0.0131 | 0.0306 | 0.0228 | 0.3312 | 0.2538 |
| Day 5 | 0.0126 | 0.0052 | 0.0144 | 0.0064 | 0.1322 | 0.0583 |
| Day 6 | 0.0152 | 0.0049 | 0.0079 | 0.0025 | 0.0677 | 0.0217 |

As shown in Table 4, for kynurenine, due to differentiation induction, an abundance (B) in a culture supernatant of the test cells is decreased relative to an abundance (A) in a culture supernatant of the control cells, and whether or not the test cells are in a differentiated state can be determined even with kynurenine alone (as described above, in the table, a value that deviates more from 1 indicates a larger change from the control cells). On the other hand, as shown in Table 27 (mTeSR1) and Table 28 (TeSR-E8), it is found that, for the ratio of kynurenine to tryptophan, which is the precursor of kynurenine, the difference from the control cells is larger as compared to the case of kynurenine alone, and this tendency becomes more prominent as the number of days of culture increases.

Comparing these results with Tables 25 and 26 which show evaluation results using a cell destructive method, it is found that, in the method of evaluating abundances in a culture supernatant of the test cells, at a stage where the number of elapsed days after the start of the differentiation induction stimulation is small, a significant difference appears between the control cells and the test cells. Further, it is found that, when the ratio of kynurenine to tryptophan rather than kynurenine alone is used as an indicator, the difference from the control cells tends to be more prominent, and thus, the differentiation state of the test cells can be evaluated with higher accuracy.

### (Omithine/Arginine)

Tables 29 and 30 show values of B/A of ornithine as a metabolic product, and values obtained by dividing B/A of ornithine by B/A of arginine which is a precursor of the ornithine cycle (urea cycle).

### [Table 29]

**Table 29 Ornithine/Arginine (mTeSR1)**

| | mTeSR1 | | | | | |
|---|---|---|---|---|---|---|
| | Endoderm Differentiation | | Mesoderm Differentiation | | Ectoderm Differentiation | |
| | Ornithine Alone | Ornithine /Arginine | Ornithine Alone | Ornithine /Arginine | Ornithine Alone | Ornithine /Arginine |
| Day 1 | 0.8824 | 0.8636 | 0.8754 | 0.9015 | 0.8191 | 0.8892 |
| Day 2 | 0.9173 | 0.8697 | 0.7791 | 0.7363 | 1.0158 | 1.0009 |
| Day 3 | 0.8515 | 0.9042 | 0.6125 | 0.5881 | 0.9221 | 1.0511 |
| Day 4 | 0.8213 | 0.6909 | 0.5843 | 0.4243 | 0.9650 | 0.9847 |
| Day 5 | 0.6869 | 0.3975 | 0.5917 | 0.3115 | 0.8816 | 0.6916 |
| Day 6 | 0.6782 | 0.3064 | 0.4975 | 0.1740 | 0.7712 | 0.4924 |

### [Table 30]

**Table 30 Ornithine/Arginine (TeSR-E8)**

| | TeSR-E8 | | | | | |
|---|---|---|---|---|---|---|
| | Endoderm Differentiation | | Mesoderm Differentiation | | Ectoderm Differentiation | |
| | Ornithine Alone | Ornithine /Arginine | Ornithine Alone | Ornithine /Arginine | Ornithine Alone | Ornithine /Arginine |
| Day 1 | 1.0432 | 1.0727 | 1.0117 | 1.0073 | 1.1216 | 1.0860 |
| Day 2 | 0.8346 | 0.8213 | 0.7999 | 0.7962 | 1.0779 | 1.0942 |
| Day 3 | 0.4761 | 0.4565 | 0.4050 | 0.3849 | 1.0404 | 1.0600 |
| Day 4 | 0.3588 | 0.3070 | 0.1664 | 0.1380 | 0.9389 | 0.9700 |
| Day 5 | 0.3590 | 0.2521 | 0.1297 | 0.0911 | 0.9076 | 0.8407 |
| Day 6 | 0.3042 | 0.2153 | 0.1443 | 0.0952 | 0.9122 | 0.8834 |

As shown in Table 5, for ornithine, due to differentiation induction, an abundance (B) in a culture supernatant of the test cells is decreased relative to an abundance (A) in a culture supernatant of the control cells, and whether or not the test cells are in a differentiated state can be determined even with ornithine alone. On the other hand, as shown in Table 29 (mTeSR1) and Table 30 (TeSR-E8), it is found that, for the endoderm differentiated cells and the mesoderm differentiated cells, for the ratio of ornithine to arginine, which is the precursor of ornithine, the difference from the control cells is larger as compared to the case of ornithine alone, and this tendency becomes more prominent as the number of days of culture increases. From these results, it is found that, by using the ratio of ornithine to arginine rather than ornithine alone as an indicator, the differentiation state of the test cells can be evaluated with higher accuracy.

### (Putrescine/Omithine)

Table 31 shows values of B/A of putrescine as a metabolic product, and values obtained by dividing B/A of putrescine by B/A of ornithine which is a precursor of arginine/proline metabolism.

### [Table 31]

**Table 31 Putrescine/Ornithine (mTeSR1)**

| | mTeSR1 | | | | | |
|---|---|---|---|---|---|---|
| | Endoderm Differentiation | | Mesoderm Differentiation | | Ectoderm Differentiation | |
| | Putrescine Alone | Putrescine /Ornithine | Putrescine Alone | Putrescine /Ornithine | Putrescine Alone | Putrescine /Ornithine |
| Day 1 | 0.9920 | 1.1242 | 0.9331 | 1.0659 | 0.8149 | 0.9948 |
| Day 2 | 1.4154 | 1.5430 | 1.3524 | 1.7357 | 0.9593 | 0.9444 |
| Day 3 | 1.1415 | 1.3406 | 0.9140 | 1.4922 | 1.0112 | 1.0966 |
| Day 4 | 1.3915 | 1.6944 | 0.7268 | 1.2439 | 1.5630 | 1.6197 |
| Day 5 | 4.3553 | 6.3406 | 0.9121 | 1.5415 | 4.6089 | 5.2277 |
| Day 6 | 4.2198 | 6.2219 | 1.1884 | 2.3885 | 4.2743 | 5.5425 |

When an mTeSR1 culture medium is used, for putrescine, due to differentiation induction, an abundance (B) in a culture supernatant of the test cells tends to increase relative to an abundance (A) in a culture supernatant of the control cells. In particular, for the endoderm differentiated cells and the ectoderm differentiated cells, this tendency becomes more prominent as the number of days of culture increases. Therefore, whether or not the test cells are in a differentiated state can be determined with putrescine alone. For the mesoderm differentiated cells, with putrescine alone, a clear trend was not observed. In contrast, by using the ratio of putrescine to ornithine, which is a precursor of putrescine, the difference from the control cells became larger as the number of days of culture increases, and, also for the mesoderm differentiated cells, whether or not the cells are in a differentiated state can be determined. It is found that, for the endoderm differentiated cells and the ectoderm differentiated cells, the difference from the control cells is larger in the case where the ratio of putrescine to ornithine, which is a precursor of putrescine, is used than in the case where putrescine alone is used, and the differentiation state of the test cells can be evaluated with higher accuracy.

## Claims

1. A cell differentiation state evaluation method, wherein
stem cells for which a differentiation state is unknown or cells differentiation-induced from pluripotent stem cells are used as test cells, and pluripotent stem cells are used as control cells, and
a differentiation state of the test cells is evaluated by comparing abundances of indicator substances in a culture supernatant of the test cells with abundances of the indicator substances in a culture supernatant of the control cells,
**characterizing in that**
the indicator substances are two or more kinds of compounds selected from a group consisting of ornithine, 2-aminoadipic acid, deoxycytidine, tryptophan, hypoxanthine, uridine and kynurenine.

2. The cell differentiation state evaluation method according to claim 1, wherein the differentiation state is evaluated based on, for each of the two or more kinds of indicator substances, whether or not a ratio of an abundance of an indicator substance in a culture supernatant of the test cells to an abundance of the indicator substance in a culture supernatant of the control cells is equal to or greater than a predetermined threshold or is equal to or less than the threshold.

3. The cell differentiation state evaluation method according to claim 1 or 2, wherein, in evaluating the differentiation state, whether or not the test cells are in a differentiated state or in an undifferentiated state is evaluated, and whether a differentiation direction of the test cells that have been evaluated as being in a differentiated state is endoderm, mesoderm, or ectoderm is evaluated.

4. The cell differentiation state evaluation method according to claim 3, wherein the evaluation is performed by analyzing change with culture time of an abundance of an indicator substance in a culture supernatant of the test cells for each of the two or more kinds of indicator substances.

5. The cell differentiation state evaluation method according to claim 3 or 4, wherein the evaluation is performed by subjecting abundances of the two or more kinds of indicator substances to multivariate analysis.

6. The cell differentiation state evaluation method according to any one of claims 1 to 5, wherein the two or more kinds of indicator substances include two or more kinds of substances selected from a group consisting of kynurenine, 2-aminoadipic acid, ornithine and deoxycytidine.

7. The cell differentiation state evaluation method according to any one of claims 1 to 6, wherein one of the two or more kinds of indicator substances is 2-aminoadipic acid.

8. The cell differentiation state evaluation method according to any one of claims 1 to 6, wherein the two or more kinds of indicator substances include a precursor and a metabolic product in an anteroposterior relation of a metabolic pathway.

9. The cell differentiation state evaluation method according to claim 8, wherein the precursor is tryptophan, and the metabolic product is kynurenine.

10. The cell differentiation state evaluation method according to any one of claims 8 or 9, wherein the evaluation is performed based on a ratio of an abundance of the precursor to an abundance of the metabolic product in a culture supernatant of the test cells.

11. A method for culturing cells comprising: a process of selecting test cells based on an evaluation result of a differentiation state of the test cells using the method according to any one of claims 1 to 10.

## Patentansprüche

1. Verfahren zur Bestimmung eines Zelldifferenzierungszustands, wobei
Stammzellen, für welche der Differenzierungszustand unbekannt ist, oder aus pluripotenten Stammzellen differenzierungsinduzierte Zellen als Testzellen verwendet werden und pluripotente Stammzellen als Kontrollzellen verwendet werden und
ein Differenzierungszustand der Testzellen durch Vergleichen von Häufigkeiten von Indikatorverbindungen in einem Kulturüberstand der Testzellen mit Häufigkeiten der Indikatorverbindungen in einem Kulturüberstand der Kontrollzellen bestimmt wird
**dadurch gekennzeichnet, dass**
die Indikatorverbindungen zwei oder mehr Arten von Verbindungen, ausgewählt aus einer Gruppe, bestehend aus Ornithin, 2-Aminoadipinsäure, Desoxycytidin, Tryptophan, Hypoxanthin, Uridin und Kynurenin, sind.

2. Verfahren zur Bestimmung eines Zelldifferenzierungszustands nach Anspruch 1, wobei der Differenzierungszustand für jede der zwei oder mehr Arten von Indikatorverbindungen auf der Grundlage bestimmt wird, ob ein Verhältnis einer Häufigkeit einer Indikatorverbindung in einem Kulturüberstand der Testzellen zu einer Häufigkeit der Indikatorverbindung in einem Kulturüberstand der Kontrollzellen gleich oder größer als ein vorbestimmter Schwellenwert oder gleich oder weniger als der Schwellenwert ist oder nicht.

3. Verfahren zur Bestimmung eines Zelldifferenzierungszustands nach einem der Ansprüche 1 oder 2, wobei beim Bestimmen des Differenzierungszustands bestimmt wird, ob die Testzellen in einem differenzierten Zustand oder in einem undifferenzierten Zustand sind oder nicht, und bestimmt wird, ob eine Differenzierungsrichtung der Testzellen, die als in einem differenzierten Zustand bestimmt wurden, endoderm, mesoderm oder ectoderm ist.

4. Verfahren zur Bestimmung eines Zelldifferenzierungszustands nach Anspruch 3, wobei die Bestimmung durch Analysieren der Änderung mit der Kultivierungszeit einer Häufigkeit einer Indikatorverbindung in einem Kulturüberstand der Testzellen für jede der zwei oder mehr Arten von Indikatorverbindungen durchgeführt wird.

5. Verfahren zur Bestimmung eines Zelldifferenzierungszustands nach Anspruch 3 oder 4, wobei die Bestimmung durch Unterziehen der Häufigkeiten der zwei oder mehr Arten von Indikatorverbindungen einer multivariaten Analyse durchgeführt wird.

6. Verfahren zur Bestimmung eines Zelldifferenzierungszustands nach einem der Ansprüche 1 bis 5, wobei die zwei oder mehr Arten von Indikatorverbindungen zwei oder mehr Arten von Verbindungen, ausgewählt aus einer Gruppe, bestehend aus Kynurenin, 2-Aminoadipinsäure, Ornithin und Desoxycytidin, beinhalten.

7. Verfahren zur Bestimmung eines Zelldifferenzierungszustands nach einem der Ansprüche 1 bis 6, wobei eine der zwei oder mehr Arten von Verbindungen 2-Aminoadipinsäure ist.

8. Verfahren zur Bestimmung eines Zelldifferenzierungszustands nach einem der Ansprüche 1 bis 6, wobei die zwei oder mehr Arten von Indikatorverbindungen einen Vorläufer und ein Stoffwechselprodukt in einer anteroposterioren Beziehung eines Stoffwechselweges beinhalten.

9. Verfahren zur Bestimmung eines Zelldifferenzierungszustands nach Anspruch 8, wobei der Vorläufer Tryptophan ist und das Stoffwechselprodukt Kynurenin ist.

10. Verfahren zur Bestimmung eines Zelldifferenzierungszustands nach einem der Ansprüche 8 oder 9, wobei die Bestimmung auf Grundlage eines Verhältnisses einer Häufigkeit des Vorläufers zu einer Häufigkeit des Stoffwechselprodukts in einem Kulturüberstand der Testzellen durchgeführt wird.

11. Verfahren zum Kultivieren von Zellen, umfassend: einen Prozess des Selektierens von Testzellen auf Grundlage eines Bestimmungsergebnisses eines Differenzierungszustands der Testzellen unter Verwendung des Verfahrens nach einem der Ansprüche 1 bis 10.

## Revendications

1. Procédé d'évaluation d'état de différenciation cellulaire, dans lequel
des cellules souches pour lesquelles un état de différenciation est inconnu ou des cellules à induction de différenciation provenant de cellules souches pluripotentes sont utilisées comme cellules d'essai, et des cellules souches pluripotentes sont utilisées comme cellules témoins, et
un état de différenciation des cellules d'essai est évalué en comparant des abondances de substances indicatrices dans un surnageant de culture des cellules d'essai à des abondances des substances indicatrices dans un surnageant de culture des cellules témoins,
**caractérisé en ce que**
les substances indicatrices sont deux types ou plus de composés sélectionnés parmi un groupe constitué de l'omithine, l'acide 2-aminoadipique, la désoxycytidine, le tryptophane, l'hypoxanthine, l'uridine et la kynurénine.

2. Procédé d'évaluation d'état de différenciation cellulaire selon la revendication 1, dans lequel l'état de différenciation est évalué, pour chacun des deux types ou plus de substances indicatrices, en fonction du fait ou non qu'un rapport d'une abondance d'une substance indicatrice dans un surnageant de culture des cellules d'essai sur une abondance de la substance indicatrice dans un surnageant de culture des cellules témoins soit supérieur ou égal à un seuil prédéterminé ou soit inférieur ou égal au seuil.

3. Procédé d'évaluation d'état de différenciation cellulaire selon la revendication 1 ou 2, dans lequel, lors de l'évaluation de l'état de différenciation, il est évalué si les cellules d'essai sont dans un état différencié ou dans un état non différencié ou non, et il est évalué si une direction de différenciation des cellules d'essai qui ont été évaluées comme étant dans un état différencié est l'endoderme, le mésoderme ou l'ectoderme.

4. Procédé d'évaluation d'état de différenciation cellulaire selon la revendication 3, dans lequel l'évaluation est réalisée en analysant un changement de la durée de culture d'une abondance d'une substance indicatrice dans un surnageant de culture des cellules d'essai pour chacun des deux types ou plus de substances indicatrices.

5. Procédé d'évaluation d'état de différenciation cellulaire selon la revendication 3 ou 4, dans lequel l'évaluation est réalisée en soumettant des abondances des deux types ou plus de substances indicatrices à une analyse multivariée.

6. Procédé d'évaluation d'état de différenciation cellulaire selon l'une quelconque des revendications 1 à 5, dans lequel les deux types ou plus de substances indicatrices incluent deux types ou plus de substances sélectionnées parmi un groupe constitué de la kynurénine, l'acide 2-aminoadipique, l'omithine et la désoxycytidine.

7. Procédé d'évaluation d'état de différenciation cellulaire selon l'une quelconque des revendications 1 à 6, dans lequel un des deux types ou plus de substances indicatrices est l'acide 2-aminoadipique.

8. Procédé d'évaluation d'état de différenciation cellulaire selon l'une quelconque des revendications 1 à 6, dans lequel les deux types ou plus de substances indicatrices incluent un précurseur et un produit métabolique dans une relation antéropostérieure d'une voie métabolique.

9. Procédé d'évaluation d'état de différenciation cellulaire selon la revendication 8, dans lequel le précurseur est le tryptophane et le produit métabolique est la kynurénine.

10. Procédé d'évaluation d'état de différenciation cellulaire selon l'une quelconque des revendications 8 ou 9, dans lequel l'évaluation est réalisée en fonction d'un rapport d'une abondance du précurseur sur une abondance du produit métabolique dans un surnageant de culture des cellules d'essai.

11. Procédé de mise en culture de cellules comprenant : un processus de sélection de cellules d'essai basé sur un résultat d'évaluation d'un état de différenciation des cellules d'essai utilisant le procédé selon l'une quelconque des revendications 1 à 10.
